# EUROPÄISCHE PATENTSCHRIFT

(11) **EP 3 777 550 B1**
(45) Veröffentlichungstag und Bekanntmachung des Hinweises auf die Patenterteilung: **30.09.2026**
(21) Anmeldenummer: 19191953.9
(22) Anmeldetag: 15.08.2019
(51) Int. Cl.: A23G 1/02, A23G 1/00, C13B 10/00

(54) **VERFAHREN ZUR GEWINNUNG EINER ZUCKERHALTIGEN SUBSTANZ AUS EINER KAKAOFRUCHT UND VERWENDUNG DER ZUCKERHALTIGEN SUBSTANZ BEI DER HERSTELLUNG VON SCHOKOLADE**
METHOD FOR OBTAINING A SUGAR-CONTAINING SUBSTANCE FROM A COCOA FRUIT AND USE OF THE SUGAR-CONTAINING SUBSTANCE IN THE PRODUCTION OF CHOCOLATE
PROCÉDÉ DE PRODUCTION D'UNE SUBSTANCE CONTENANT DU SUCRE À PARTIR D'UNE CABOSSE ET UTILISATION DE LA SUBSTANCE CONTENANT DU SUCRE DANS LA FABRICATION DE CHOCOLAT

(43) Veröffentlichungstag der Anmeldung: 17.02.2021
(73) Patentinhaber: Alfred Ritter GmbH & Co. KG, 71111 Waldenbuch (DE)
(72) Erfinder: HOPPE, Tim, 72766 Reutlingen (DE); BACHMEIR, Johann, 72770 Reutlingen (DE); ARETZ, Jan, 72760 Reutlingen (DE)
(74) Vertreter: Wasmuth, Rolf

(56) Entgegenhaltungen:
- EP-A1- 3 498 102
- WO-A1-2017/044610
- CH-A5- 622 028
- DE-A1- 2 230 444
- FR-A1- 2 828 379
- ANVOH ET AL: "Production and characterization of juice from mucilage of cocoa beans and its transformation into marmalade.", PAKISTAN JOURNAL OF NUTRITION, vol. 8, no. 2, 31 December 2009 (2009-12-31), pages 129 - 133, XP055368951, DOI: 10.3923/pjn.2009.129.133
- DATABASE WPI Week 201535, Derwent World Patents Index; AN 2015-23504A

## Beschreibung

Die Erfindung betrifft ein Verfahren zur Gewinnung einer zuckerhaltigen Substanz aus einer Kakaofrucht nach dem Oberbegriff des Anspruchs 1 sowie eine ausschließlich aus weiterverarbeiteten Kakaofrüchten hergestellte Schokolade.

Die in einer Kakaofrucht befindlichen, meist in fünf Längsreihen angeordneten Kakaobohnen sind bei der Ernte etwa 2 cm bis 4 cm lang und 1,2 cm bis 2 cm breit sowie in ihrer Form oval oder elliptisch. Die Kakaobohnen sind in einer süßen Schicht Fruchtfleisch eingebettet, der sogenannten Pulpe, die zur Fermentation genutzt wird.

Nach dem Ernten werden die Kakaobohnen zusammen mit dem anhaftenden oder losen Fruchtfleisch aufgehäuft, um den Fermentationsprozess einzuleiten. Beim Öffnen der Kakaofrucht werden Fruchtfleisch eigene Enzyme freigesetzt, durch welche das Fruchtfleisch verflüssigt wird. Das süße Fruchtfleisch gerät innerhalb der aufgehäuften Menge unter Druck, wodurch das Abfließen der Flüssigkeit begünstigt wird. In einer ersten, der anaeroben Phase wird die Fermentation durch Abbau des im Fruchtfleisch enthaltenen Zuckers durch Hefen eingeleitet. Durch die Enzyme und durch die Hefen wird die Pulpe verflüssigt und trennt sich von den Schalen der Kakaobohnen. Die abfließenden Flüssigkeiten werden entsorgt oder versickern im Erdreich. Nun kann Luft in die entstehenden Hohlräume zwischen den Kakaobohnen eindringen. Der in der abfließenden Flüssigkeit enthaltene Zucker wird durch die Hefen in Alkohol umgewandelt. In einer folgenden aeroben Phase wird der Alkohol von Essigsäurebakterien zu Essigsäure oxidiert, wobei die Temperatur der Kakaobohnen auf 45° bis 55° ansteigen kann.

Bekannt ist, die Kakaobohnen mit dem Fruchtfleisch in Fermentationsgebinde, wie beispielsweise Fermentierkörbe oder dgl. einzufüllen, wobei durch Zwischenräume in den Fermentationsgebinden anfallende Flüssigkeiten abfließen können. Das Durchmischen der Kakaobohnen zur Zufuhr von Luft erfolgt durch Umfüllen in andere Fermentationsgebinde.

Bei der Haufenfermentation werden die Kakaobohnen z. B. auf einem Bretterboden zu Haufen aufgeschüttet und abgedeckt. Für eine ausreichende Luftzufuhr müssen die Haufen regelmäßig umgeschichtet werden.

Aus der WO 2017/044610 A1 ist ein Verfahren sowie eine Anlage zur Herstellung von Kakaosirup bekannt.

Aus der EP 3 498 102 A1 ist ein Schokoladenprodukt und dessen Herstellungsverfahren bekannt, wobei sämtliche Zutaten für ein solches Schokoladenprodukte aus einer Kakaofrucht entnommen sind.

Der Erfindung liegt die Aufgabe zugrunde, eine Kakaofrucht intensiv zu verwerten und auch die bei der Schichtung der Kakaobohnen anfallenden Flüssigkeiten zu verwerten.

Die Aufgabe wird nach den Merkmalen des Anspruchs 1 gelöst.

Die bei der Vorbereitung zur Fermentation anfallenden Flüssigkeiten werden gesammelt, gereinigt und zu einer zuckerhaltigen Substanz aufkonzentriert. Damit wird ein weiterer Bestandteil der Kakaofrucht verwendet. Die Kakaofrucht wird umfassend genutzt.

Die Kakaobohnen werden zusammen mit dem Fruchtfleisch in einer vorgegebenen Menge und/oder in einem vorgegebenen Gewicht aufgehäuft. Dabei wirkt auf die unteren Schichten die Gewichtskraft der darüber aufgehäuften Kakaobohnen, wodurch das Fruchtfleisch gepresst wird und einen Teil seiner Flüssigkeit abgibt. Vorteilhaft ist vorgesehen, die Kakaobohnen mit dem Fruchtfleisch mehrfach umzuschichten, so dass ein Großteil der Kakaobohnen mit dem Fruchtfleisch von oberen Lagen in untere Lagen verlagert wird, wodurch auch dieses Fruchtfleisch gepresst und zur Abgabe eines Kakaosaftes angeregt wird.

Vorteilhaft ist, dass die ablaufende Flüssigkeit nur innerhalb von bis zu sechs Stunden nach Öffnen der Kakaofrucht gesammelt wird. Um die Umwandlung des Zuckers in der Flüssigkeit zu vermeiden, ist diese noch vor Beginn der Fermentierung weiterzuverarbeiten. Vorzugsweise wird die Flüssigkeit pasteurisiert. Dadurch kann die Fermentierung sowie der damit verbundene Zuckerverlust der Flüssigkeit vermieden werden.

Es ist vorteilhaft vorgesehen, dass das in der Flüssigkeit enthaltene Pektin abgebaut wird. Vorzugsweise wird die Flüssigkeit zum Entfernen von Trübstoffen filtriert. Um der zuckerhaltigen Substanz den säuerlichen Geschmack zu entnehmen, werden vorteilhaft die in der Flüssigkeit enthaltenen Säuren entzogen.

Nach dem Reinigen der ablaufenden Flüssigkeit, insbesondere durch mechanische Reinigung und/oder Filterung, sowie dem Abbau der Säuren wird die gereinigte Flüssigkeit zu einem Dicksaft konzentriert. Dies kann durch Reduktion des Wassergehaltes des Dicksaftes erfolgen. Hierzu wird die Flüssigkeit vorzugsweise eingedampft. Der Gesamtzuckergehalt des dann konzentrierten Dicksaftes beträgt vorzugsweise mindestens 60% der Gesamtmasse des Dicksaftes. Vorzugsweise liegt der Gesamtzuckergehalt des Dicksaftkonzentrates in einem Bereich von 60% bis 70% der Gesamtmasse des Dicksaftes. Der Dicksaft ist über einen Trägerstoff zu trocknen. In anderen Worten ist die in dem Dicksaft verbleibende Restfeuchte mittels Trocknung mit Hilfe eines Trägerstoffes zu entfernen. Es ist vorgesehen, dass als Trägerstoff entweder Kakaomasse oder Kakaopulver einsetzbar sind.

Es kann vorteilhaft sein, die Deaktivierung von Pektin und/oder das Abbauen der in der gesammelten Flüssigkeit enthaltenen Säure erst dann auszuführen, wenn die Flüssigkeit bereits zu einem Dicksaft aufkonzentriert ist.

Um eine maximale Menge der aus dem Haufen austretenden Flüssigkeit zu gewährleisten, ist vorgesehen, auf die aufgehäuften Kakaobohnen und das aufgehäufte Fruchtfleisch zusätzlich eine Druckkraft auszuüben. Dies kann durch die Bestimmung der Größe eines Haufens unter Nutzung der Schwerkraft bzw. des Gewichts des Haufens ausgeführt werden. Alternativ oder zusätzlich kann vorgesehen sein, auf die aufgehäuften Kakaobohnen zusätzlich eine Druckkraft auszuüben, z. B. eine maschinelle Druckkraft oder das Aufsetzen eines Druckstempels unter Nutzung der Schwerkraft.

Mit der erfindungsgemäßen zuckerhaltigen Substanz wird insbesondere eine Schokolade produziert. Die zuckerhaltige Substanz kann aber auch nach Trocknung Kakaopulver oder dgl. Erzeugnissen zugesetzt werden. Eine Schokolade enthält zumindest eine ausgewählte Menge Kakao, eine zugesetzte Menge an Fett und eine zugesetzte zuckerhaltige Substanz. Es ist vorgesehen, dass das Fett Kakaobutter und die zuckerhaltige Substanz ein aus der Pulpe der Kakaofrucht gewonnener Kakaozucker, insbesondere Dicksaft nach dem vorstehend beschriebenen Verfahren ist.

Es ist eine Schokolade geschaffen, deren wesentliche Bestandteile, insbesondere deren alle Bestandteile aus Kakaofrüchten gewonnen sind.

Weitere Merkmale der Erfindung ergeben sich aus den weiteren Ansprüchen, der Beschreibung und der Zeichnung, in der eine Vorrichtung zum Sammeln von Kakaosaft sowie ein Verfahren zur Gewinnung einer zuckerhaltigen Substanz schematisch dargestellt und nachfolgend im Einzelnen beschrieben ist. Es zeigen:
- Fig. 1: in schematischer Ansicht einen Aufnahmebehälter zum Fermentieren und/oder Trocknen von Kakaobohnen,
- Fig. 2: eine Draufsicht auf die Vorrichtung nach Fig. 1,
- Fig. 3: eine Seitenansicht der Vorrichtung nach Fig. 1,
- Fig. 4: in schematischer Darstellung das erfindungsgemäße Verfahren zur Gewinnung einer zuckerhaltigen Substanz.

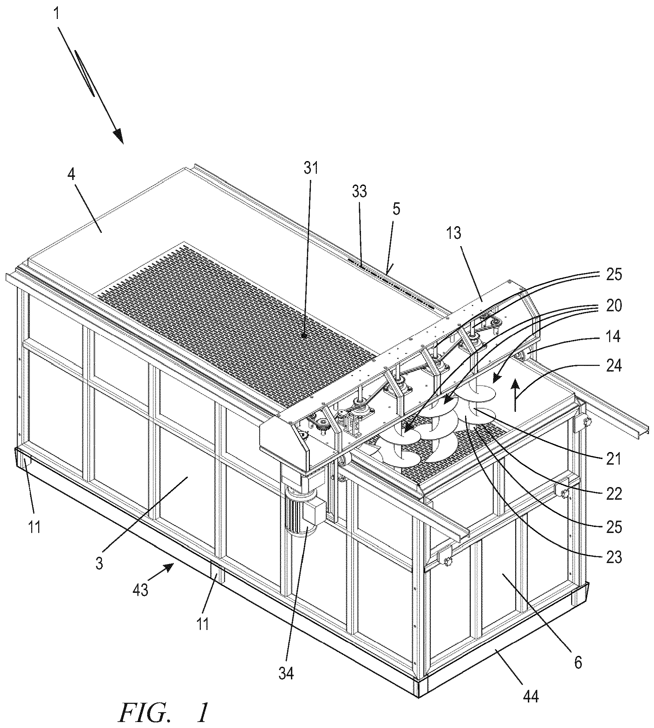

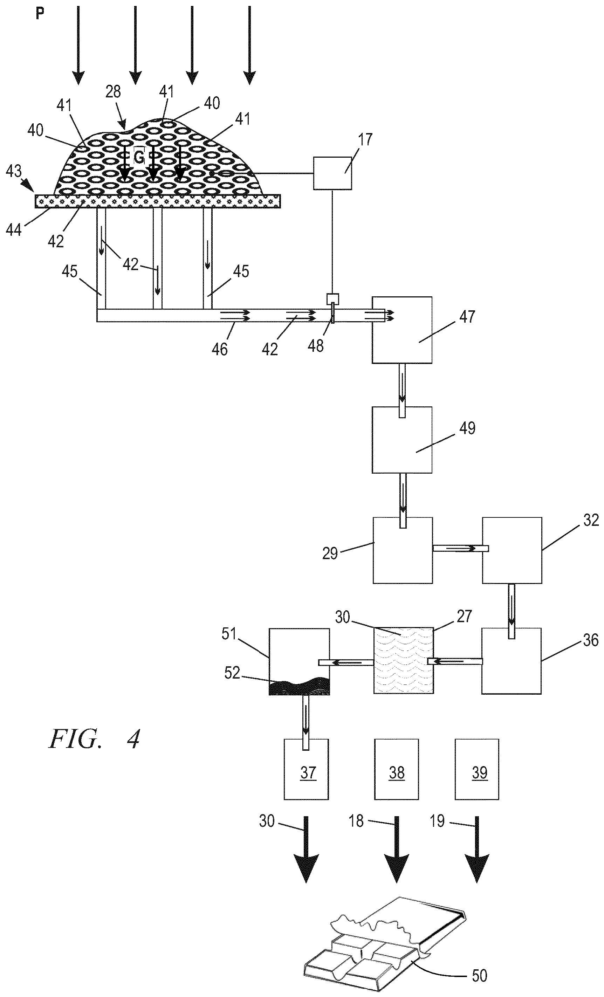

Der in den Figuren 1 bis 3 dargestellte Behälter 1 dient zum Fermentieren und/oder Trocknen von Kakaobohnen 40 (Fig.4) aus dem Inneren einer Kakaofrucht. Eine Kakaobohne 40 ist etwa 2 cm bis 4 cm lang und 1,2 cm bis 2 cm breit und in der Form oval oder elliptisch. Die Kakaobohnen 40 sind von einer dünnen, süßen Schicht Fruchtfleisch 41 umgeben, der sogenannten Pulpe, auf dem sich nach dem Aufbrechen der Kakaofrüchte eine Vielzahl von Mikroorganismen ansiedeln. Durch die Mikroorganismen durchläuft das die Kakaobohnen 40 umgebende Fruchtfleisch 41 einen Fermentationsprozess, wodurch die Farbe und der Geruch der Kakaobohnen 40 entstehen. Sobald der Fermentationsprozess beginnt, wandeln Hefen den Zucker des Fruchtfleisches 41 in Alkohol und Kohlendioxyd um. Bakterien oxidieren den so gewonnenen Alkohol, wobei unter aeroben Bedingungen Essigsäure und in geringen Mengen auch Milchsäure entstehen kann. Das Fruchtfleisch 41 wird nach und nach abgebaut, wobei die Bakterien aktiv bleiben, bis der Fermentationsprozess beendet ist. Sämtliche Mikroorganismen, die die Fermentation bewirken, stammen aus der natürlichen Umgebung oder können durch Inokulation hinzugefügt werden. Nach Abschluss der Fermentation werden die Kakaobohnen 40 getrocknet.

Im weiteren Verlauf des Prozesses werden die Bohnen gereinigt, von ihren Schalen abgetrennt und geröstet.

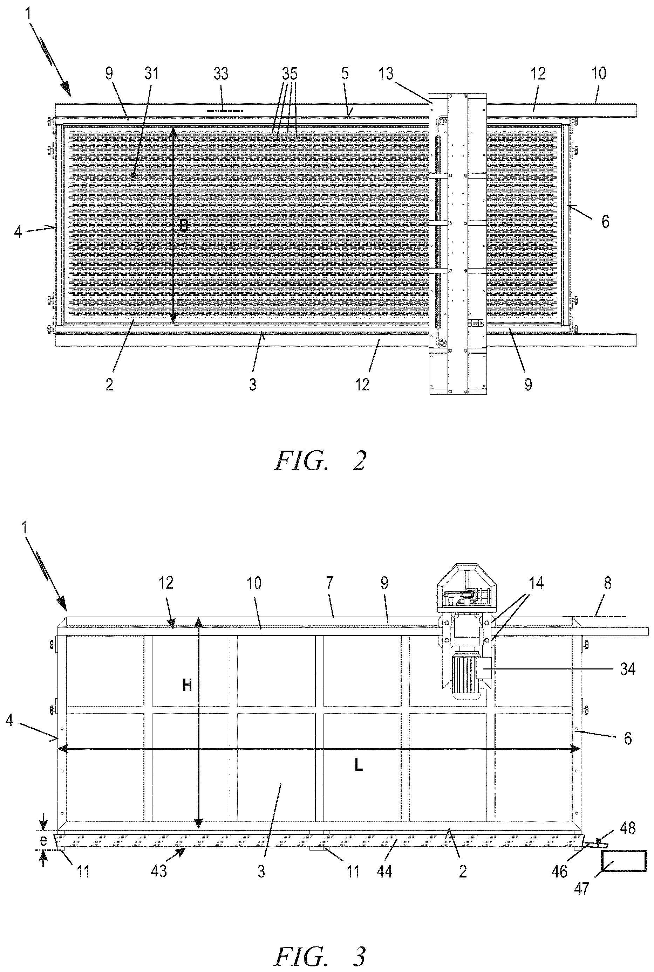

Der in den Figuren 1 bis 3 gezeigte trogartige Behälter 1 weist einen Boden 2 und den Boden 2 umfassende Gehäusewände auf. Die Gehäusewände bestehen aus langen Seitenwänden 3, 5 und schmalen Stirnwänden 4 und 6. Der Behälter 1 hat in Draufsicht gemäß Fig. 3 eine im Wesentlichen rechteckige Form mit einer inneren Höhe H sowie einer inneren Breite B. Ferner hat der Behälter eine innere Länge L (Fig. 3).

Im Ausführungsbeispiel haben die Gehäusewände eine Höhe H von etwa 50 cm, wobei die Stirnwände 4 und 6 eine Breite B von 120 cm aufweisen. Die Seitenwände 3, 5 haben eine Länge von etwa 300 cm. Die Maße gelten nur als Beispiel; die Maße können nach Bedarf geändert werden.

Am oberen Längsrand 9 der Seitenwände 3 und 5 sind Träger 10 angeordnet, die Führungsbahnen 12 für einen Wagen 13 bilden. Der Wagen 13 ist durch Führungsrollen 14 geführt, wobei die Träger 10 Schienen für die Führungsrollen 14 bilden.

An dem Wagen 13 ist mindestens ein Mischorgan 20 vorgesehen, welches aus einer Welle 21 mit einem an der Welle 21 vorgesehenen Mischglied 22 ausgebildet ist. Vorzugsweise ist als Mischglied 22 auf der zentralen Welle 21 eine Mischschnecke 23 ausgebildet, wobei die Mischschnecke 23 bevorzugt als Wendel vorgesehen ist. Die Wendel verläuft unterbrechungsfrei von einem angetriebenen Ende 25 der Welle 21 bis zu einem freien Ende 26 der Welle. Die Wendel hat eine vorgegebene Steigung.

Quer zum Fahrweg 33 des Wagens 13 sind über die Breite B des Bodens 2 mehrere Mischorgane 20 vorgesehen. Im gezeigten Ausführungsbeispiel nach Fig. 1 sind vier Mischorgane 20 in einer Reihe nebeneinander liegend angeordnet.

Die Mischorgane 20 erstrecken sich jeweils aufrecht vom Boden 2, wobei die Wellen 21 der Mischorgane 20 bevorzugt lotrecht zum Boden 2 liegen.

Der Wagen 13 hat ferner einen Antrieb 34, der bevorzugt durch Antriebsrollen gebildet ist. Die nebeneinander liegenden Mischorgane 20 decken die gesamte Breite B des Behälters 1 ab. So wird gewährleistet, dass durch eine einfache Hin- und Her-Bewegung längs des Fahrweges 33 jede Stelle des Innenraums des Behälters 1 zu erreichen ist; die aktive Mischzone der Mischorgane erreicht jeden Ort innerhalb des Behälters 1.

Im Boden 2 des Behälters 1 ist eine Vielzahl von Durchbrüchen 35 vorgesehen, über die einerseits in den Behälter 1 Luft, z. B. Umgebungsluft, einströmen kann und andererseits Flüssigkeit 42 (Fig. 4) aus dem Behälter 1 ablaufen kann. Die aus den angehäuften Kakaobohnen 40 ablaufende Flüssigkeit 42 wird insbesondere in einer Wanne 44, einer Auffangschale oder dgl. Sammelbehälter 43 aufgefangen.

Zweckmäßig steht der Behälter 1 auf Füßen 11, so dass zwischen dem Boden 2 des Behälters 1 und einer Standfläche ein Abstand e ausgebildet ist. Der Sammelbehälter 43 ist derart gestaltet, dass er unterhalb des Bodens 2 innerhalb des Abstandes e positioniert werden kann.

Bevorzugt bildet der Boden 2 einen Gitterrost 31. Die im Boden 2 vorgesehenen Durchbrüche 35 sind bevorzugt aus Löchern gebildet. Hierzu ist vorzugsweise ein Lochblech als Boden 2 zu verwenden. Der Durchmesser der Löcher beträgt weniger als 10 mm, insbesondere etwa 5 mm. Es kann auch in einer alternativen Ausführung zweckmäßig sein, anstelle des Lochbleches einen Spaltenboden vorzusehen. In den Behälter 1 eingefüllte Kakaobohnen 40 werden so vom Gitterrost 31 sicher gehalten, während die Flüssigkeit 42 behinderungsfrei abfließen kann. Über die Durchbrüche 35 des Gitterrostes 31 kann ferner Luft zum Trocknen, insbesondere konditionierte Luft eingeblasen werden.

In den Behälter 1 eingefüllte Kakaobohnen 40 mit Fruchtfleisch 41 werden durch die Mischorgane 20 durchgemischt, wobei aufgrund der Förderrichtung 24 der Mischorgane 20 vom Boden 2 aufwärts in Richtung auf die Ebene 8 des Gehäuserandes 7 die dem Boden 2 nächstliegenden Kakaobohnen 40 nach oben gefördert werden. Unten liegende Kakaobohnen 40 und das unten liegende Fruchtfleisch 41 gelangen nach oben und oben liegende Kakaobohnen 40 sowie das oben liegende Fruchtfleisch 41 gelangen nach unten. Die Gewichtskraft G der übereinanderliegenden Kakaobohnen sowie dem übereinander liegenden Fruchtfleisch 41 wirkt so auf die gesamte in den Behälter 1 eingefüllte Menge an Kakaobohnen 40, so dass allein durch die Gewichtskraft G ein effektives Abpressen der zu gewinnenden Flüssigkeit 42 erzielt ist. Um die Gewinnung der Flüssigkeit 42 aus der Pulpe zeitlich zu beschleunigen, kann auch eine Vorrichtung mit einer Presse vorgesehen sein. Bei einer solchen Vorrichtung wird die Pulpe mittels eines Stempels gegen ein Gitterrost gepresst, so dass die Flüssigkeit aus der Pulpe unter erhöhtem Druck ablaufen kann.

Ist der Fermentationsprozess zu Ende, wird Umgebungsluft, die vorzugsweise konditioniert ist, z. B. vorgewärmt sein kann, über den Boden 2 in den Behälter 1 eingeblasen, um die Kakaobohnen 40 zu trocknen. Über die Vielzahl der vorgesehenen Durchbrüche 35 im Gitterrost 31 ist ein guter Luftzutritt zu dem gesamten Behälterinhalt gewährleistet. Die Durchbrüche 35 sind über die gesamte Fläche des Bodens 2 verteilt, wodurch eine gleichmäßige Luftzufuhr an jeder Stelle des Behälters 1 zu erzielen ist.

Das erfindungsgemäße Verfahren ist in Fig. 4 in einer schematischen Übersicht dargestellt. Die z. B. auf einem Haufen 28 aufgehäufte Menge an Kakaobohnen 40 zusammen mit dem anhaftenden und dem losen Fruchtfleisch 41 liegt auf einem Gitterrost 31 gemäß Fig. 2. Unterhalb des Gitterrostes 31 ist ein Sammelbehälter 43 in Form einer Wanne 44 vorgesehen, welche über Ablaufrohre 45 und ein Sammelrohr 46 in einen Vorratsbehälter 47 mündet. Das Sammelrohr 46 ist mit einer Sperreinrichtung 48 versehen, welche vorteilhaft von einer Steuereinheit 17 gesteuert ist. In einer alternativen Ausführung kann es zweckmäßig sein, unterhalb des Gitterrostes 31 ein schräg geneigtes Blech vorzusehen, an welchem die Flüssigkeit in ein Sammelgefäß abläuft.

Der Fermentationsprozess beginnt bereits mit Öffnung der Kakaofrucht. In etwa sechs Stunden nach Öffnung der Kakaofrucht hat die Fermentation eine Intensität erreicht, bei welcher bereits größere Mengen an Zucker abgebaut werden. Daher wird höchstens sechs Stunden lang nach Öffnung der Kakaofrucht die Flüssigkeit aus der Pulpe gesammelt.

Durch Anhäufen der Kakaobohnen 40 zusammen mit dem Fruchtfleisch 41 in einem Haufen 28 wirkt eine Gewichtskraft G insbesondere auf die untere Schicht der Kakaobohnen 40. Dadurch fließt ein erster Anteil an aus dem Fruchtfleisch 41 ausgepresster Flüssigkeit 42 über die Ablaufrohre 45 in den Vorratsbehälter 47 oder über das schräg geneigte Blech in das Sammelgefäß.

Werden die Kakaobohnen 40 mit dem Fruchtfleisch 41 in einen Behälter 1 gemäß den Figuren 1 und 3 eingefüllt und durch die Mischorgane 20 in Förderrichtung 24 durchgemischt, so gelangen während einer Mischzeit ein Großteil bis alle Kakaobohnen 40 mit dem anhaftenden Fruchtfleisch 41 auch nach unten und liegen insbesondere nahe des Gitterrostes 31. Daher wirkt auf einen Großteil bis auf alle der im Behälter 1 eingefüllten Kakaobohnen 40 mit dem Fruchtfleisch 41 im Laufe der Mischzeit die Gewichtskraft G der Behälterfüllung. Bereits in den ersten sechs Stunden nach Öffnung der Kakaofrucht, in welchen lediglich ein nur vernachlässigbar geringer Anteil an Zucker in der Flüssigkeit abgebaut ist, fließt somit eine große Menge an Flüssigkeit 42 ab, welche über den Sammelbehälter 43 gesammelt und dem Vorratsbehälter 47 zugeführt ist. Es kann vorteilhaft sein, auf die angehäuften Kakaobohnen 40 mit dem anhaftenden Fruchtfleisch 41 eine Druckkraft P auszuüben, um die Ausbeute an Flüssigkeit 42 vor dem Beginn des Fermentationsprozesses zu erhöhen.

Die im Vorratsbehälter 47 gesammelte Flüssigkeit 42 wird, um die Fermentierung bzw. die Umwandlung des Zuckers in der Flüssigkeit zu vermeiden, in einer Stabilisierungsstufe 49 pasteurisiert. Da das Endprodukt, also die zuckerhaltige Substanz möglichst viel Zucker enthalten soll, muss der Zucker in der Flüssigkeit stabilisiert werden. Die Pasteurisierung kann mittels Röhrenwärmetauscher oder mittels eines Pasteur erfolgen. Anschließend wird die Flüssigkeit wieder abgekühlt, um die thermische Belastung zu reduzieren.

In einem nächsten Verfahrensschritt, der Pektinstufe 29, wird die Flüssigkeit von Pektin befreit. Hierzu werden entsprechende Enzyme, nämlich Pektinasen, der Flüssigkeit hinzugegeben. Die Pektinasen bauen das Pektin ab bzw. deaktivieren das Pektin in der Flüssigkeit. Darauf folgt das Filtrieren der nun pektinfreien Flüssigkeit in einer Filtrationsstufe 32. Die Flüssigkeit durchläuft eine Membrantrennanlage, die beispielsweise eine Polymermembran in Form einer Hohlfaser umfasst. Auch der Einsatz anderer Membranen, wie beispielsweise einer keramischen Membran, kann zweckmäßig sein. Hat die Flüssigkeit die Membrantrennanlage durchlaufen, sind die Verunreinigungen in Form von Trübstoffen aus der Flüssigkeit gefiltert.

In diesem Verfahrensabschnitt ist die Flüssigkeit befreit von Trübstoffen und von Pektin. Allerdings beinhaltet die Flüssigkeit vielerlei verschiedene Säuren, wie beispielsweise Citronen-, Apfel-, Essig-, Oxal- und Milchsäure. Auch Galacturonsäure ist nach dem Abbau von Pektin in der Flüssigkeit enthalten. Diese Säuren werden in dem Verfahrensschritt der Entsäuerungsstufe 36 abgebaut, um der Flüssigkeit ihren säuerlichen Geschmack zu entnehmen. Hierzu wird ein schwach basischer Anionentauscher eingesetzt, an dem die in der Flüssigkeit vorhandenen Säuren gebunden werden.

Im Verfahrensschritt 27, nämlich der Verdampferstufe, wird die Flüssigkeit 42 zu einem Dicksaft 30 aufkonzentriert. Dabei wird die Flüssigkeit 42, die in diesem Verfahrensschritt ein entsäuertes Saftkonzentrat ist, mittels eines Vakuumverdampfers eingedampft. Der Vakuumverdampfer kann beispielsweise ein Rotationsverdampfer sein. Das Saftkonzentrat befindet sich in einem in etwa 40°C temperierten Behälter unter Vakuum, so dass das Saftkonzentrat bei entsprechend niedriger Temperatur bereits verdampft. Die Temperatur des Behälters kann mittels eines Wasserbades eingestellt werden, wobei der Behälter im Wasserbad rotiert, damit das in dem Behälter befindliche Saftkonzentrat gleichmäßig auf 40°C temperiert wird. Der beim Eindampfen entstehende Dicksaft 30 weist einen Gesamtzuckergehalt auf, der vorzugsweise mindestens 60% der Gesamtmasse des Dicksaftes 30 entspricht. Vorzugsweise liegt der Gesamtzuckergehalt des Dicksaftkonzentrates in einem Bereich von 60% bis 70% der Gesamtmasse des Dicksaftes 30.

Der Dicksaft 30 enthält noch eine gewisse Restfeuchte, die die Verwendung des Dicksaftes 30 als Mittel zum Süßen erschwert. Daher wird in einem weiteren Verfahrensschritt, nämlich der Trocknungsstufe 51 der Dicksaft 30 getrocknet. Hierfür werden Trägerstoffe eingesetzt. Als Trägerstoffe werden entweder Kakaomasse oder Kakaopulver eingesetzt, wodurch der daraus entstehende Kakaozucker 52 vollständig aus der Kakaofrucht gewonnen ist.

In Fig. 4 ist ferner dargestellt, dass über eine erste Dosiervorrichtung 37 der Kakaozucker 52 einer Schokoladenfertigung zugeführt wird. Über eine zweite Dosiervorrichtung 38 wird aus der Kakaobohne gewonnene Kakaobutter 18 und über eine dritte Dosiervorrichtung 39 die gewünschte Menge an Kakao 19 zugeführt. Die Schokoladenfertigung kann in bekannter Weise erfolgen; als Ergebnis wird eine Schokolade 50 erhalten, die mit einer zuckerhaltigen Substanz gemäß vorstehendem Verfahren gefertigt ist. Rohrzucker, Traubenzucker, Rübenzucker oder Zuckerersatzstoffe sind vermieden. Als zuckerhaltige Substanz kann ausschließlich der Kakaozucker 52 verwendet werden.

Die gefertigte Schokolade 50 enthält zumindest eine ausgewählte Menge Kakao 19, die über die Dosiervorrichtung 39 zugeführt ist. Ferner enthält die Schokolade als Fettanteil Kakaobutter 18, die über die Dosiervorrichtung 38 zugeführt ist. Als Mittel zum Süßen wird die aus der Pulpe der Kakaofrucht gewonnene zuckerhaltige Substanz, insbesondere umfassend Kakaozucker 52, über die Dosiervorrichtung 37 zugeführt. Die gefertigte Schokolade 50 besteht somit ausschließlich aus den aus der Kakaofrucht gewonnenen Stoffen. Alle Bestandteile der Schokolade sind aus Kakaofrüchten gewonnen.

Die ablaufende Flüssigkeit wird innerhalb von sechs Stunden nach Öffnen der Kakaofrucht gesammelt, um einen maximalen Zuckergehalt in der Flüssigkeit zu gewährleisten. Dadurch kann die gewonnene Flüssigkeitsmenge gesteigert und so die Ausbeute an Kakaozucker 52 erhöht werden.

## Patentansprüche

1. Verfahren zur Gewinnung einer zuckerhaltigen Substanz aus einer Kakaofrucht zur Verwendung in der Lebensmittelindustrie, insbesondere zur Verwendung bei der Herstellung von Schokolade (50), wobei die Kakaofrucht aufgebrochen und die vom Fruchtfleisch (41) umhüllten Kakaobohnen (40) zusammen mit dem Fruchtfleisch (41) entnommen werden, wobei die entnommenen Kakaobohnen (40) zusammen mit dem Fruchtfleisch (41) aufgehäuft werden,
wobei die aus den aufgehäuften Kakaobohnen (40) ablaufende Flüssigkeit (42) gesammelt, gereinigt und zu einer zuckerhaltigen Substanz aufkonzentriert wird, wobei die Flüssigkeit (42) zu einem Dicksaft (30) konzentriert wird,
**dadurch gekennzeichnet, dass** der Dicksaft (30) mittels eines Trägerstoffes getrocknet wird, wobei der Trägerstoff Kakaomasse oder Kakaopulver ist.

2. Verfahren nach Anspruch 1,
**dadurch gekennzeichnet, dass** die Kakaobohnen (40) zusammen mit dem Fruchtfleisch (41) in einer vorgegebenen Menge und/oder Gewicht aufgehäuft werden.

3. Verfahren nach Anspruch 1,
**dadurch gekennzeichnet, dass** die ablaufende Flüssigkeit (42) nur innerhalb von bis zu sechs Stunden nach Öffnen der Kakaofrucht gesammelt wird.

4. Verfahren nach einem der Ansprüche 1 bis 3,
**dadurch gekennzeichnet, dass** die ablaufende Flüssigkeit (42) pasteurisiert wird.

5. Verfahren nach einem der Ansprüche 1 bis 4,
**dadurch gekennzeichnet, dass** das in der Flüssigkeit (42) enthaltene Pektin abgebaut wird.

6. Verfahren nach einem der Ansprüche 1 bis 5,
**dadurch gekennzeichnet, dass** die Flüssigkeit (42) zum Entfernen von Trübstoffen filtriert wird.

7. Verfahren nach einem der Ansprüche 1 bis 6,
**dadurch gekennzeichnet, dass** die in der Flüssigkeit (42) enthaltenen Säuren entzogen werden.

8. Verfahren nach Anspruch 7,
**dadurch gekennzeichnet, dass** die in der Flüssigkeit vorhandenen Säuren an einem schwach basischen Anionentauscher gebunden werden.

9. Verfahren nach einem der Ansprüche 1 bis 8,
**dadurch gekennzeichnet, dass** die Flüssigkeit (42) eingedampft wird.

10. Verfahren nach Anspruch 9,
**dadurch gekennzeichnet, dass** der Gesamtzuckergehalt des eingedampften Dicksaftes (30) mindestens 60% beträgt.

11. Verfahren nach einem der Ansprüche 1 bis 10,
**dadurch gekennzeichnet, dass** auf die aufgehäuften Kakaobohnen (40) eine Druckkraft (G, P) ausgeübt wird.

12. Verfahren nach einem der Ansprüche 1 bis 11,
**dadurch gekennzeichnet, dass** die aufgehäuften Kakaobohnen (40) gepresst werden.

## Claims

1. Method for obtaining a sugar-containing substance from a cocoa fruit for use in the food industry, in particular for use in the production of chocolate (50), wherein the cocoa fruit is broken open and the cocoa beans (40) enveloped by the fruit pulp (41) are removed together with the fruit pulp (41), wherein the removed cocoa beans (40) are heaped up together with the fruit pulp (41), wherein the liquid (42) flowing off from the heaped-up cocoa beans (40) is collected, purified and concentrated to form a sugar-containing substance, wherein the liquid (42) is concentrated to form a thick juice (30), **characterized in that** the thick juice (30) is dried by means of a carrier material, wherein the carrier material is cocoa mass or cocoa powder.

2. Method according to claim 1,
**characterized in that** the cocoa beans (40) are heaped up together with the fruit pulp (41) in a predetermined amount and/or weight.

3. Method according to claim 1,
**characterized in that** the flowing-off liquid (42) is collected only within up to six hours after opening of the cocoa fruit.

4. Method according to one of claims 1 to 3,
**characterized in that** the flowing-off liquid (42) is pasteurized.

5. Method according to one of claims 1 to 4,
**characterized in that** the pectin contained in the liquid (42) is degraded.

6. Method according to one of claims 1 to 5,
**characterized in that** the liquid (42) is filtered for removing turbid substances.

7. Method according to one of claims 1 to 6,
**characterized in that** the acids contained in the liquid (42) are removed.

8. Method according to claim 7,
**characterized in that** the acids present in the liquid are bound to a weakly basic anion exchanger.

9. Method according to one of claims 1 to 8,
**characterized in that** the liquid (42) is evaporated.

10. Method according to claim 9,
**characterized in that** the total sugar content of the evaporated thick juice (30) is at least 60%.

11. Method according to one of claims 1 to 10,
**characterized in that** a compressive force (G, P) is exerted on the heaped-up cocoa beans (40).

12. Method according to one of claims 1 to 11,
**characterized in that** the heaped-up cocoa beans (40) are pressed.

## Revendications

1. Procédé pour l'obtention d'une substance contenant du sucre à partir d'un fruit de cacao destinée à être utilisée dans l'industrie alimentaire, en particulier destinée à être utilisée lors de la fabrication de chocolat (50), le fruit de cacao étant ouvert et les fèves de cacao (40) enveloppées par la pulpe (41) étant prélevées avec la pulpe (41), les fèves de cacao (40) prélevées étant entassées avec la pulpe (41),
le liquide (42) s'écoulant des fèves de cacao (40) entassées étant recueilli, purifié et concentré en une substance contenant du sucre, le liquide (42) étant concentré en un sirop épais (30),
**caractérisé en ce que** le sirop épais (30) est séché au moyen d'une substance porteuse, la substance porteuse étant de la masse de cacao ou de la poudre de cacao.

2. Procédé selon la revendication 1,
**caractérisé en ce que** les fèves de cacao (40) sont entassées avec la pulpe (41) dans une quantité et/ou un poids prédéterminé.

3. Procédé selon la revendication 1,
**caractérisé en ce que** le liquide (42) s'écoulant est recueilli uniquement dans un délai allant jusqu'à six heures après l'ouverture du fruit de cacao.

4. Procédé selon l'une quelconque des revendications 1 à 3,
**caractérisé en ce que** le liquide (42) s'écoulant est pasteurisé.

5. Procédé selon l'une quelconque des revendications 1 à 4,
**caractérisé en ce que** la pectine contenue dans le liquide (42) est dégradée.

6. Procédé selon l'une quelconque des revendications 1 à 5,
**caractérisé en ce que** le liquide (42) est filtré afin d'éliminer des substances troubles.

7. Procédé selon l'une quelconque des revendications 1 à 6,
**caractérisé en ce que** les acides contenus dans le liquide (42) sont éliminés.

8. Procédé selon la revendication 7,
**caractérisé en ce que** les acides présents dans le liquide sont liés à un échangeur d'anions faiblement basique.

9. Procédé selon l'une quelconque des revendications 1 à 8,
**caractérisé en ce que** le liquide (42) est évaporé.

10. Procédé selon la revendication 9,
**caractérisé en ce que** la teneur totale en sucre du sirop épais (30) évaporé est d'au moins 60%.

11. Procédé selon l'une quelconque des revendications 1 à 10,
**caractérisé en ce qu'**une force de pression (G, P) est exercée sur les fèves de cacao (40) entassées.

12. Procédé selon l'une quelconque des revendications 1 à 11,
**caractérisé en ce que** les fèves de cacao (40) entassées sont pressées.
